# EUROPEAN PATENT APPLICATION

(11) **EP 3 878 376 A1**
(43) Date of publication of application: **15.09.2021**
(21) Application number: 21161901.0
(22) Date of filing: 10.03.2021
(51) Int. Cl.: A61B 17/04, A61B 17/062

(54) **SURGICAL STITCHING DEVICE**

(30) Priority: 11.03.2020 US 202062988121 P; 29.12.2020 US 202017136629
(71) Applicant: Covidien LP, Mansfield, MA 02048 (US)
(72) Inventor: AVVLN, Srinivasa Aurthy Aravalli, 534211 Tanuku (IN); GUTTI, Ravi Sekhar, 500049 Hyderabad (IN); MANDULA, Rajanikanth, 5000040 Hyderabad (IN)
(74) Representative: Maschio & Soames IP Ltd

(57) **Abstract**

A surgical stitching device includes a fixed jaw, a rotating jaw, and a transfer mechanism. The rotating jaw is movable in relation to the fixed jaw between a start position and a transfer position. The transfer mechanism includes a first electromagnetic coil supported on the fixed jaw and a second electromagnetic coil supported on the rotating jaw. The first and second electromagnetic coils can be independently energized to transfer the needle between the fixed jaw and the rotating jaw.

## Description

### CROSS-REFERENCE TO RELATED APPLICATION

This application claims the benefit of and priority to U.S. Provisional Patent Application No. 62/988,121, filed on March 11, 2020, the entire disclosure of which is incorporated by reference herein.

### FIELD

The technology is generally related to surgical stitching devices and, more particularly, to surgical stitching devices including structure for transferring a needle between jaws of the stitching device.

### BACKGROUND

Surgical stitching devices are known to perform a variety of surgical procedures. Typically, surgical stitching devices include a rotating jaw, a fixed jaw, a transferring mechanism, and a needle that supports a suture. The needle includes a curved body that has tapered ends that are configured to pierce tissue. The rotating jaw and the fixed jaw each include a bore that is positioned to receive one of the tapered ends of the needle as the rotating jaw is moved in relation to the fixed jaw. The transferring mechanism is provided to secure the needle alternatingly to one of the fixed jaw and the rotating jaw. The stitching device also includes a switch for activating the transferring mechanism.

In known stitching devices, the switch is manually activated by a clinician and includes a blade that is movable between retracted and advanced positions into engagement with the needle to secure the needle onto one of the fixed jaw and the rotating jaw. The needle often includes a notch that receives the blade to enhance securement. The need for a clinician to properly operate the transferring device to ensure that the needle is transferred between the jaws is necessary for a successful suturing procedure.

A continuing need exists for a surgical stitching device with a less complex transferring mechanism.

### SUMMARY

This disclosure is directed to a surgical stitching device that includes a fixed jaw, a rotating jaw, and a transfer mechanism. The rotating jaw is movable in relation to the fixed jaw between a start position and a transfer position. The transfer mechanism includes a first electromagnetic coil supported on the fixed jaw and a second electromagnetic coil supported on the rotating jaw. The first and second electromagnetic coils can be independently energized to transfer the needle between the fixed jaw and the rotating jaw.

One aspect of the disclosure is directed to a stitching device including a handle assembly, an elongate body, and a tool assembly. The handle assembly includes a switch and an actuator. The elongate body extends distally from the handle assembly and includes a distal end portion. The tool assembly is supported on the distal end portion of the elongate body and includes a fixed jaw, a rotating jaw, and a needle. The rotating jaw is movable in relation to the fixed jaw from a start position to a transfer position in response to operation of the actuator. The fixed jaw supports a first electromagnetic coil and the rotating jaw supports a second electromagnet coil. Each of the first and second electromagnetic coils can be independently energized via operation of the switch to secure the needle to one of the fixed jaw or the rotating jaw.

Another aspect of the disclosure is directed to a tool assembly including a fixed jaw, a rotating jaw, and a needle. The rotating jaw is movable in relation to the fixed jaw from a start position to a transfer position. The fixed jaw supports a first electromagnetic coil and the rotating jaw supports a second electromagnet coil. Each of the first and second coils can be independently energized to secure the needle to one of the fixed jaw or the rotating jaw.

In aspects of the disclosure, the first electromagnetic coil defines a first bore, the second electromagnetic coil defines a second bore, and the needle includes a first tapered end and a second tapered end, wherein in the start position, the first tapered end is positioned within the first bore or the second tapered end is positioned within the second bore, and in the transfer position, the first tapered end is positioned within the first bore and the second tapered end is positioned within the second bore.

Another aspect of the disclosure is directed to a stitching device including a handle assembly, an elongate body, a drive assembly, and a tool assembly. The handle assembly includes a switch and an actuator. The elongate body extends distally from the handle assembly. The drive assembly includes a drive rod having a distal end, a cam support member supported on the distal end of the drive rod, and a cam member that is supported on the cam support member. The drive rod is movable between retracted and advanced positions in response to operation of the actuator. The tool assembly includes a fixed jaw, a rotating jaw, and a needle. The rotating jaw includes a helical cam slot that receives the cam member such that operation of the actuator causes movement of the rotating jaw in relation to the fixed jaw from a start position to a transfer position. The fixed jaw supports a first electromagnetic coil and the rotating jaw supports a second electromagnet coil. Each of the first and second electromagnetic coils can be energized via operation of the switch to secure the needle to one of the fixed jaw or the rotating jaw.

In some aspects of the disclosure, the needle includes a curved body.

In certain aspects of the disclosure, the needle is formed at least in part from a ferromagnetic material.

In aspects of the disclosure, the first and second electromagnetic coils, when energized, create a magnetic field to retain the needle within a respective one of the bores of the first or second electromagnetic coils.

In some aspects of the disclosure, the first jaw defines a first bore that receives the first electromagnetic coil and the second jaw defines a second bore that receives the second electromagnetic coil.

In certain aspects of the disclosure, the first and second bores of the first and second electromechanical coils define axes that are transverse to the longitudinal axis of the elongate body.

In aspects of the disclosure, the first electromagnetic coils supports a first plunger and the second electromagnetic coil supports a second plunger, wherein the first and second electromagnetic coils are operable to move the first and second plungers, respectively, from a retracted position to an advanced position into engagement with the needle.

In some aspects of the disclosure, each of the first and second plungers defines a notch that is configured to receive the needle to retain the needle in engagement with a respective one of the respective first and second jaws.

In certain aspects of the disclosure, the first electromagnetic coil defines a first bore and the second electromagnetic coil defines a second bore, wherein the first and second bores of the first and second electromagnetic coils define axes that are parallel to the longitudinal axis of the elongate body.

In aspects of the disclosure, each of the first and second electromagnetic coils is coupled to the switch by wires.

In some aspects of the disclosure, the actuator is positioned to engage the switch such that operation of the actuator activates the switch.

Other features of the disclosure will be appreciated from the following description.

### BRIEF DESCRIPTION OF DRAWINGS

Various aspects of the disclosure are described herein below with reference to the drawings, wherein:
FIG. 1 is a perspective view of a surgical stitching device including exemplary aspects of the disclosure;
FIG. 2 is an enlarged view of the indicated area of detail shown in FIG. 1;
FIG. 2A is an exploded view of the distal portion of the surgical stitching device shown in FIG. 1 including a tool assembly;
FIG. 3 is a side perspective view of an electromagnetic coil of the surgical stapling device shown in FIG. 1;
FIG. 4 is an exploded view of a fixed jaw of the tool assembly of the surgical stitching device shown in FIG. 2;
FIG. 5 is a side perspective view of the tool assembly of the surgical stitching device shown in FIG. 1 in a start position with a needle secured to a rotating jaw of the tool assembly as the tool assembly is manipulated to penetrate tissue with the needle;
FIG. 6 is a side perspective cutaway view of a portion of a handle assembly of the surgical stitching device shown in FIG. 1 illustrating a rocker switch in a first position to energize the electromagnetic coil in the rotating jaw;
FIG. 7 is a side perspective view of the tool assembly shown in FIG. 5 as the rotating jaw of the tool assembly is rotated from the start position towards the fixed jaw to advance the needle and a suture attached to the needle through tissue and into engagement with the fixed jaw;
FIG. 8 is a side perspective cutaway view of the portion of a handle assembly shown in FIG. 6 with the rocker switch in a second position to energize the electromagnetic coil in the fixed jaw and secure the needle to the fixed jaw;
FIG. 9 is a side perspective view of the tool assembly shown in FIG. 5 as the rotating jaw of the tool assembly is rotated back towards the start position to separate the rotating jaw from the needle;
FIG. 10 is a side perspective view of the tool assembly shown in FIG. 5 with the rotating jaw in the start position and the fixed jaw manipulated to advance the needle back through tissue;
FIG. 11 is a side perspective view of the tool assembly shown in FIG. 5 as the rotating jaw of the tool assembly is rotated from the start position back towards the fixed jaw into engagement with the needle;
FIG. 12 is a side perspective cutaway view of a portion of the handle assembly of the surgical stitching device shown in FIG. 1 with the rocker switch in the first position to energize the electromagnetic coil in the rotating jaw;
FIG. 13 is a side perspective view of the tool assembly shown in FIG. 11 as the rotating jaw of the tool assembly is rotated with the needle back to the start position to pull the needle and the suture attached to the needle back through the tissue;
FIG. 14 is a top view of the handle assembly of the surgical stitching device including other exemplary aspects of disclosure with the handle assembly in a non-actuated position;
FIG. 15 is a top view of the handle assembly of the surgical stitching device shown in FIG. 14 with the handle assembly in an actuated position; and
FIG. 16 is a side perspective view of the distal portion of other exemplary aspects of the disclosed surgical stitching device.

### DETAILED DESCRIPTION

The disclosed surgical stitching device will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "clinician" is used generally to refer to medical personnel including doctors, nurses, and support personnel.

The disclosed surgical stitching device shown generally as stitching device 10 includes a handle assembly 12, an elongate body 14, and a tool assembly 16. The handle assembly 12 includes a housing 18, an actuator 20, and a switch 22. In aspects of the disclosure, the actuator 20 includes spaced triggers 24 that are coupled to the housing 18 about pivot members 26. The triggers 24 pivot in relation to the housing 18 and each other to actuate the tool assembly 16 as described in further detail below. It is envisioned that the actuator 20 can assume a variety of configurations and can include a single trigger, lever, or the like. The elongate body 14 defines a longitudinal axis "X" and includes a tubular housing 28 having a proximal end 28a and a distal end 28b. The proximal end 28a of the tubular housing 28 of the handle assembly 12 is secured to the housing 18 of the handle assembly 12 and the distal end 28b of the tubular housing 28 of the elongate body 14 supports the tool assembly 16 at a position spaced from the handle assembly 12.

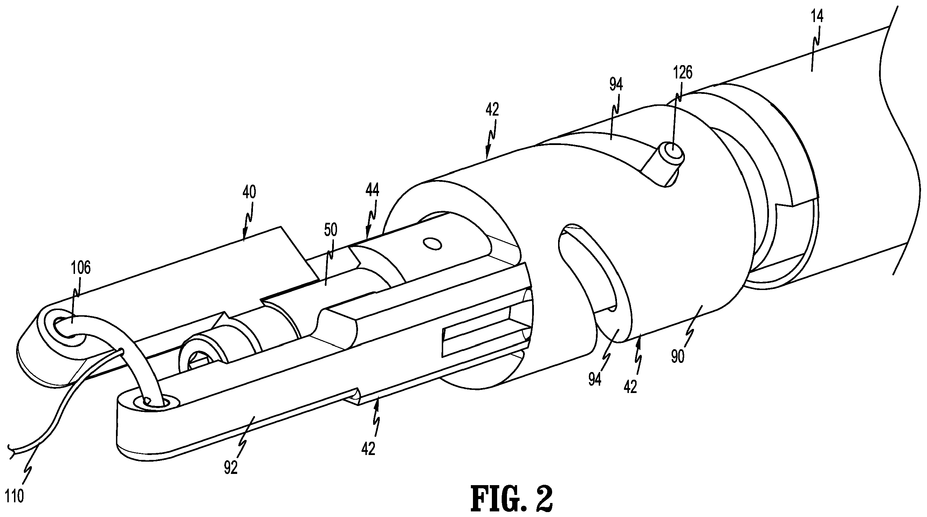

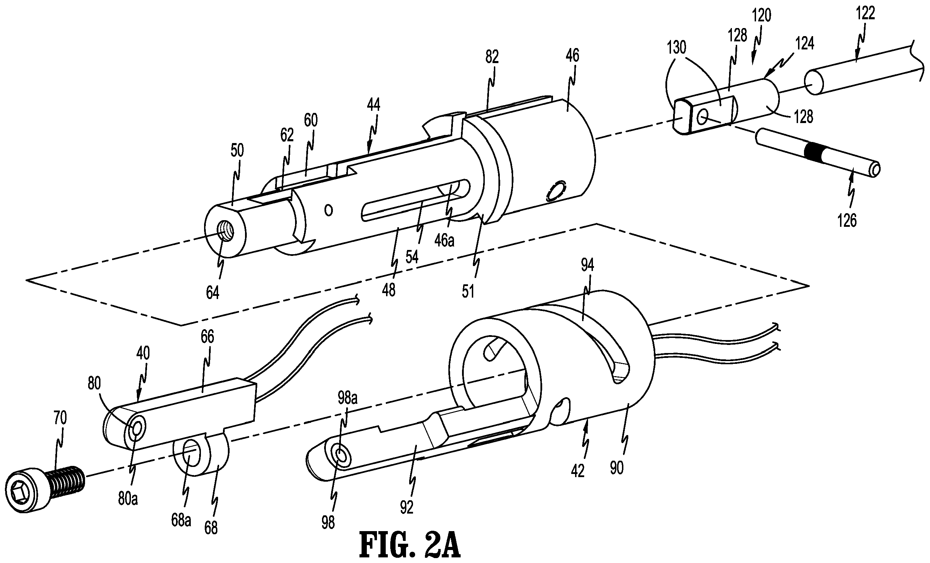

FIGS. 2-4 illustrate the tool assembly 16 of the stapling device 10 which includes a fixed jaw 40, a rotating jaw 42, and a fixed jaw base 44 that supports the fixed jaw 40. The fixed jaw base 44 includes a proximal hub portion 46, a cylindrical central body portion 48, and a distal jaw support portion 50. The central body portion 48 is tubular and has a diameter that is smaller than the diameter of the proximal hub portion 46 to define a shoulder 51 between the proximal hub portion 46 and the central body portion 48. The central body portion 48 defines diametrically opposed longitudinal slots 54, and a through channel 56 that extends along the central body portion 48 though the proximal hub portion 46. The central body portion 48 has a distal portion that defines a cutout 60 having a flat base 62 that extends distally onto the jaw support portion 50. The hub portion 46 is also tubular and defines a bore 46a that extends into the central body portion 48. In some aspects of the disclosure, the jaw support portion 50 includes a longitudinal threaded bore 64.

The fixed jaw 40 of the stitching device 10 includes a body 66 having a flat support surface 68 that is received on the flat base 62 (FIG. 2A) of the cutout 60. The body 66 supports a coupling member 68 in the form of a loop that defines an opening 68a that is aligned with the threaded bore 64 of the jaw support portion 50 of the fixed jaw base 44. In aspects of the disclosure, the opening 68 receives a threaded bolt 70 which is threaded into the threaded bore 64 of the fixed jaw base 44 to secure the fixed jaw 40 to the fixed jaw base 44. Alternately, it is envisioned that a variety of different securement devices and techniques could be used to secure the fixed jaw 40 to the fixed jaw base 44. It is also envisioned that the fixed jaw 40 and the fixed jaw base 44 could be integrally and monolithically formed.

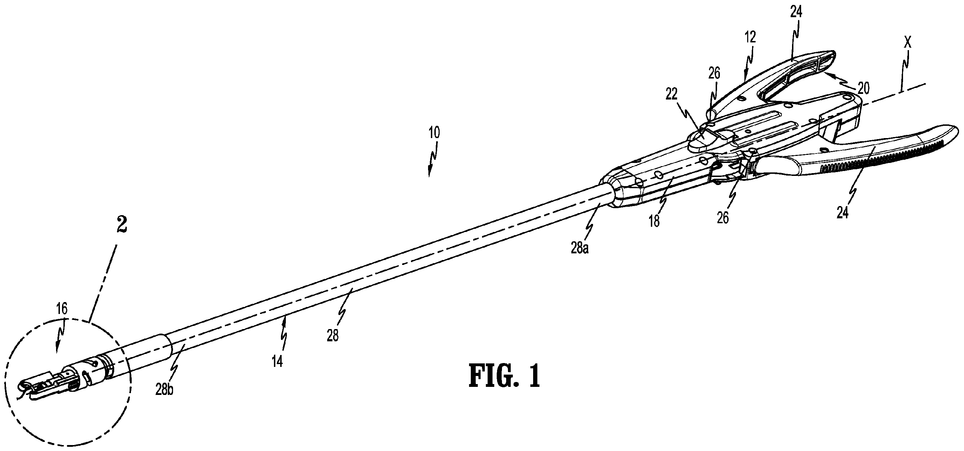

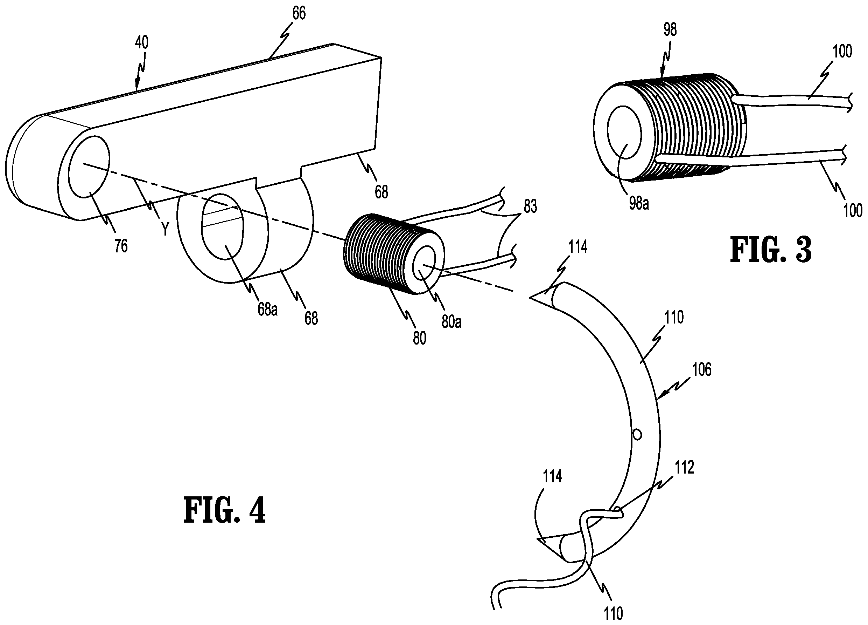

The fixed jaw 40 defines a cylindrical bore 76 (FIG. 4) that defines an axis that is transverse to a longitudinal axis "X" of the elongate body 14 (FIG. 1). The cylindrical bore 76 receives an electromagnetic coil 80 that is coupled to wires 83 that extend proximally from the electromagnetic coil 80 through channels (not shown) in the fixed jaw 40 and a channel 82 formed in the fixed jaw base 44 into the tubular housing 28 of the elongate body 14. The wires 83 are coupled to the switch 22 of the handle assembly 12. Although not shown, the handle assembly 12 includes a power source, e.g., a battery pack, to selectively deliver power to the electromagnetic coil 80 via the switch 22 and the wires 83.

The rotating jaw 42 includes a proximal hub portion 90 and a jaw portion 92. The proximal hub portion 90 is tubular and defines a helical cam slot 94. The proximal hub portion 90 is rotatably supported about the central body portion 44 of the fixed jaw base 44 such that the proximal end of the proximal hub portion 90 of the rotating jaw 42 abuts the shoulder 51 of the fixed jaw base 44. The jaw portion 92 extends distally from the proximal hub portion 90 and defines a cylindrical bore 96 (FIG. 2A) that defines an axis that is transverse to a longitudinal axis "X" of the elongate body 14 (FIG. 1). The cylindrical bore 96 receives an electromagnetic coil 98 that is coupled to wires 100 that extend proximally from the electromagnetic coil 98 through channels (not shown) in the rotating jaw 44 and a channel (not shown) formed in the rotating jaw 42 into the tubular housing 28 of the elongate body 14. The wires 100 are coupled to the switch 22 of the handle assembly 12 (FIG. 1). As described above, the handle assembly 12 includes a power source, e.g., a battery pack, to selectively deliver power to the electromagnetic coil 98 via the switch 22 and the wires 100.

The tool assembly 16 includes a needle 106 and a suture 108. The needle 106 includes a curved body 110 that defines a bore 112 that receives the suture 110 to secure one end of the suture to the needle 106. The curved body 110 of the needle 106 has ends 114 that are tapered to pierce tissue. The needle 106 is formed from, or coated with, a ferromagnetic material and is dimensioned to be alternately received within bores 80a, 98a of the electromagnetic coils 80, 98 as described in further detail below.

FIG. 2A illustrates the distal portion of a drive assembly 120 of the stitching device 10. The drive assembly 120 includes a drive rod 122, a cam support member 124 supported on a distal end of the drive rod 122, and a cam member 126 that is supported on the cam support member 124. The drive rod 122 has a longitudinal axis that is coaxial with the longitudinal axis "X" of the elongate body 14 and extends through the elongate body 14. The drive rod 122 has a proximal end (not shown) that is operably associated with the handle assembly 12 (FIG. 1) such that actuation of the triggers 24, i.e., pivotable movement of the triggers 24 towards the housing 18 of the handle assembly 12, effects longitudinal movement of the drive rod 122 within the elongate body 14. The distal end of the drive rod 122 is secured to the cam support member 124 using any suitable fastening technique such that longitudinal movement of the drive rod 122 effects longitudinal movement of the cam support member 124. It is envisioned that the cam support member 124 can be integrally or monolithically formed with the drive rod 122. In aspects of the disclosure, the cam support member 124 includes a cylindrical body 128 having diametrically disposed flats 130. The body 128 defines a through bore 132 that extends between the flats 130.

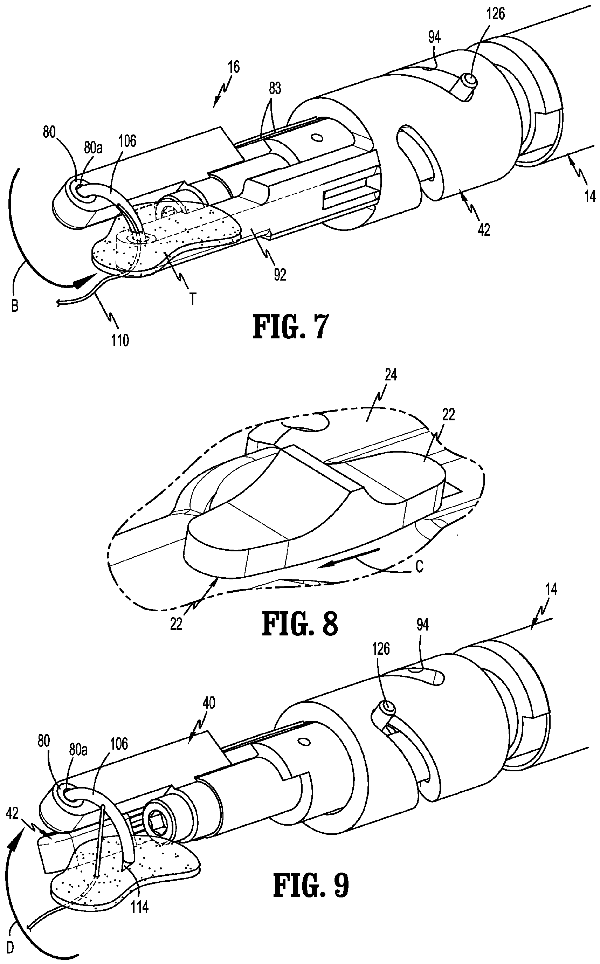

The cam member 126 extends through the through bore 132 in the body 128 in a direction transverse to the longitudinal axis "X" of the elongate body 14. The cam member 126 is secured to the cam support member 124 using any suitable securement technique including threading, welding crimping or the like. When the stitching device 10 is assembled, the cam support member 124 is received within the bore 46a defined within the fixed jaw base 44 and the cam member 134 extends transversely outwardly from the cam support member 124, through the diametrically opposed longitudinal slots 54 in the central body portion 48 of the fixed jaw base 44, and through the helical cam slot 94 in the rotating jaw 42 FIG. 2). As described in further detail below, when the triggers 24 are compressed and moved towards the housing 18 of the handle assembly 12, the drive rod 122 of the drive assembly 120 is moved from an advanced position to a retracted position within the elongate body 14 to move the cam member 126 from an advanced position to a retracted position within the helical cam slot 94 of the proximal hub portion 90 of the rotating jaw 42. As the cam member 126 moves through the helical cam slot 94 in the rotating jaw 42, engagement between the cam member 126 and walls defining the cam slot 94 causes the rotating jaw 42 to rotate in the direction of arrow "B" in FIG. 7 about the central body portion 48 of the fixed jaw 40 from a start position to a transfer position.

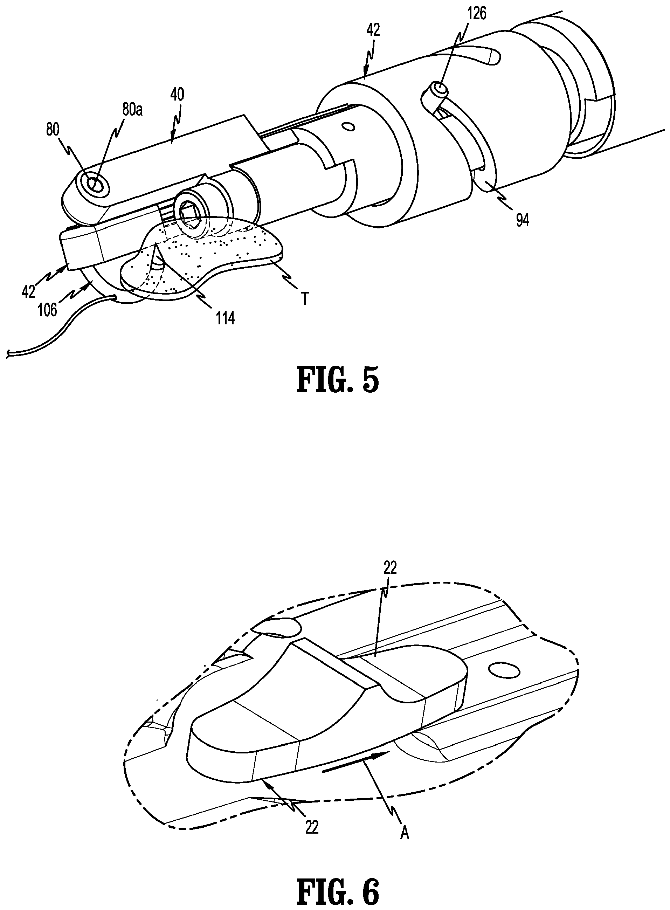

FIGS. 5 and 6 illustrate portions of the stitching device 10 when the stitching device 10 is in the start position. More specifically, FIG. 5 illustrates the distal portion of the stitching device 10 with the tool assembly 16 in the start position and FIG. 6 illustrates the switch 22 on the handle assembly 12 when the tool assembly 16 is in the start position. In the start position, one of the tapered ends 114 of the needle 106 is supported within the electromagnetic coil 98 of the jaw portion 92 of the rotating jaw 42 and the other tapered end 114 of the needle 106 of the rotating jaw 42 is spaced from the fixed jaw 40 of the tool assembly 16. In the start position, the switch 22 (FIG. 1) on the handle assembly 12 is moved to a first position in the direction of arrow "A" in FIG. 6 to energize the electromagnetic coil 98 on the rotating jaw 42. When the electromagnetic coil 98 (FIG. 4) on the rotating jaw 42 is energized, a magnetic field is created on the rotating jaw 42 to secure the tapered end 114 of the needle 106 that is positioned within the bore 98a of the electromagnetic coil 98 onto the rotating jaw 42.

When the tool assembly 15 of the stitching device 10 is in the start position (FIG. 5), the stitching device 10 can be manipulated to move the tapered end 114 of the needle 106 that is exposed through tissue "T". As shown, the suture 110 is secured to the needle 106.

When the spaced triggers 24 of the handle assembly 12 are compressed towards the housing 18 of the handle assembly 12, the drive rod 122 of the drive assembly 120 is moved from its advanced position towards its retracted position within the elongate body 14 to move the cam member 126 of the drive assembly 120 from its advanced position towards its retracted position within the helical cam slot 94 of the proximal hub portion 90 of the rotating jaw 42. As the cam member 126 moves through the helical cam slot 94 in the rotating jaw 42, engagement between the cam member 126 and walls defining the cam slot 94 causes the rotating jaw 42 to rotate in the direction of arrow "B" in FIG. 7 to move the rotating jaw 342 in relation to the fixed jaw 40 from the start position to a transfer position. As the rotating jaw 42 moves in relation to the fixed jaw 40, the needle 106 moves through the tissue "T" and into the bore 80a of the electromagnetic coil 80 on the fixed jaw 40. When the needle 106 moves into the bore 80a of the electromagnetic coil 80, the switch 22 on the handle assembly 12 can be moved by the clinician in the direction of arrow "C" in FIG. 8 to deenergize the electromagnetic coil 98 and energize the electromagnetic coil 80 to secure the needle 106 onto the fixed jaw 40. As the needle is moved through the tissue "T", the suture 110 which is attached to the needle 106 is pulled through the tissue "T".

When the triggers 24 of the handle assembly 12 are released by the clinician, a spring mechanism (not shown) in the handle assembly 12 returns the triggers 24 to their non-compressed positions. When the triggers 24 return to their non-compressed positions, the drive rod 122 of the drive assembly 120 is moved from its retracted position towards its advanced position within the elongate body 14 to move the cam member 126 of the drive assembly 120 from its retracted position towards its advanced position within the helical cam slot 94 of the proximal hub portion 90 of the rotating jaw 42. As the cam member 126 moves through the helical cam slot 94 in the rotating jaw 42, engagement between the cam member 126 and walls defining the cam slot 94 causes the rotating jaw 42 to rotate in the direction of arrow "D" in FIG. 9 back towards the start positon. As the rotating jaw 42 moves back to the start position, the rotating jaw 42 moves in relation to the fixed jaw 40 away from the exposed end 114 of the needle 106 which is supported on the fixed jaw 42.

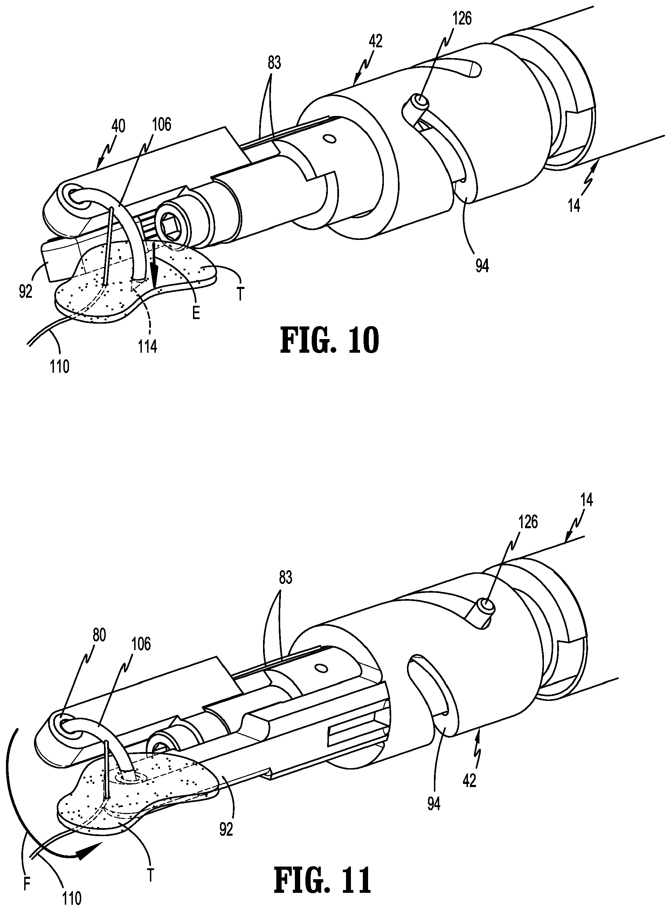

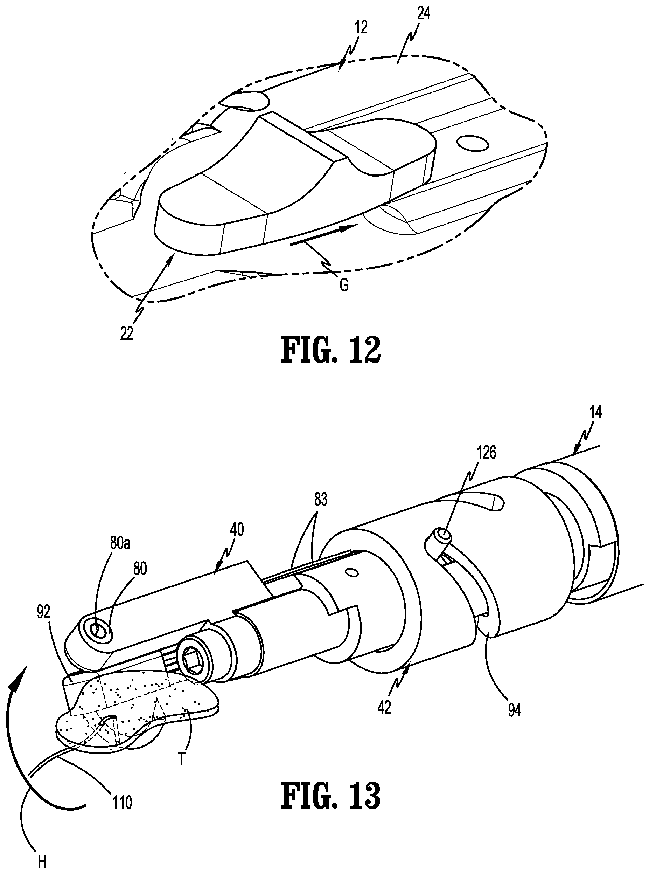

FIGS. 10 and 11 illustrate the stitching device 10 as a second stitch is placed in the tissue "T". When the needle 106 is supported on the fixed jaw 40, the stitching device 10 can be manipulated to advance the exposed end 114 of the needle 106 in the direction of arrow "E" in FIG. 10 through the tissue "T". After the exposed end 114 of the needle 106 is passed through the tissue "T", the triggers 24 of the handle assembly 12 can be once again compressed towards the housing 18 of the handle assembly 12 as described above to move the rotating jaw 42 in relation to the fixed jaw 40 in the direction of arrow "F" in FIG. 11 to reposition the end 114 of the needle 106 within the electromagnetic coil 98 on the rotating jaw 42. As illustrated in FIG. 12, the switch 22 (FIG. 1) on the handle assembly 12 can be moved in the direction of arrow "G" to energize the electromagnetic coil 98 and deenergize the coil 80 to secure the needle 106 back onto the rotating jaw 42.

When the triggers 24 of the handle assembly 12 are released by the clinician, the drive rod 122 of the drive assembly 120 is once again moved from its retracted position towards its advanced position within the elongate body 14 to move the cam member 126 of the drive assembly 120 from its retracted position towards its advanced position within the helical cam slot 94 of the proximal hub portion 90 of the rotating jaw 42. As described above, this causes the rotating jaw 42 to rotate in the direction of arrow "H" in FIG. 13 back towards the start position to move the needle 106 away from the stationary jaw 40. As the needle 106 moves away from the fixed jaw 40, the needle 106 is pulled through the tissue "T" to pull the suture 110 back through the tissue "T". This process is repeated until the tissue "T" is properly sutured.

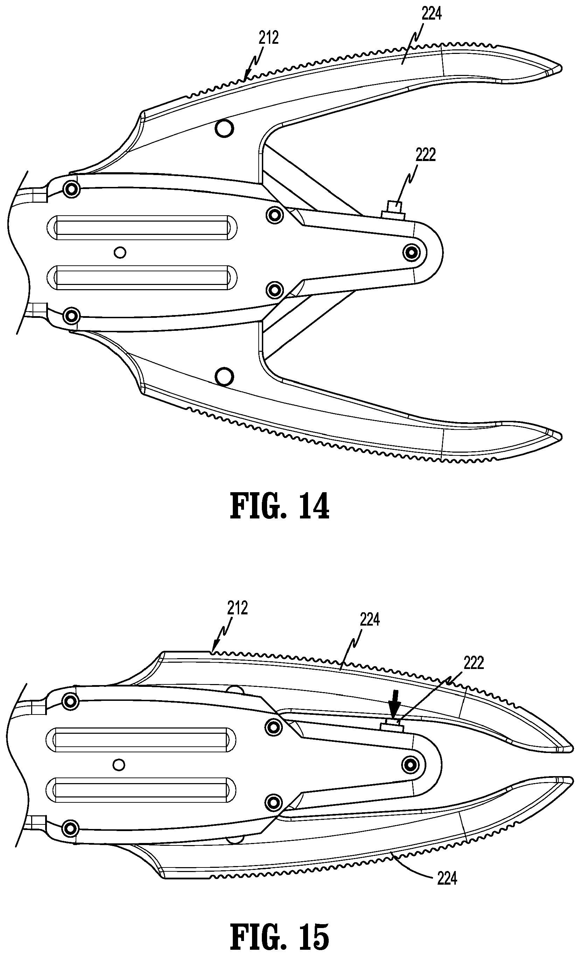

FIGS. 14 and 15 illustrate additional aspects of the disclosure in which the switch 22 (FIG. 1) of the handle assembly 12 has been changed to a depressible switch 222. The depressible switch 222 is positioned to be engaged by one of the triggers 224 of the handle assembly 212 when the triggers 224 are operated. More specifically, each time the triggers 224 are compressed towards the housing 219 of the handle assembly 212, one of the handles 224 engages the depressible switch 222 to actuate the switch 222. The switch 222 is configured such that each time the switch 222 is depressed, the power to the electromagnet coils 80, 98 is reversed, i.e., the electromagnetic coil that is energized is deenergized and the electromagnetic coil that is deenergized is energized. This allows the needle 106 (FIG. 2A) to be automatically released from one jaw and secured to the other jaw by actuating the triggers 224. Unlike the switch 22 (FIG. 1), operation of the switch 222 does not require a separate action by the clinician.

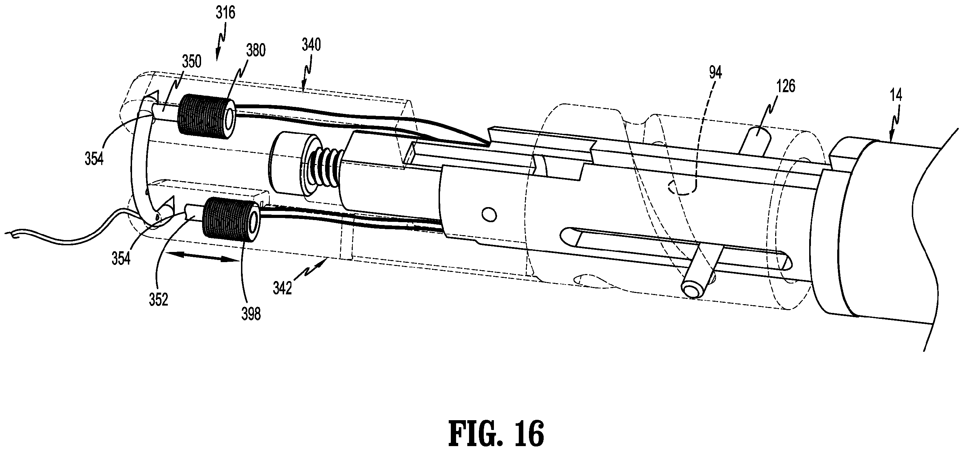

FIG. 16 illustrates other aspects of the disclosure including an alternative tool assembly shown as tool assembly 316. The tool assembly 316 can be used with the handle assembly 12 and the elongate body 14 described above in regard to stitching device 10 (FIG. 1). Accordingly, only the tool assembly 316 will be described in further detail herein.

The tool assembly 316 includes a needle 306, a fixed jaw 340, and a rotating jaw 342 that is movable in relation to the fixed jaw 340 to transfer the needle 306 between the jaws 340, 342. The fixed jaw 340 and the rotating jaw 342 are substantially similar in configuration and operation to the jaws 40, 42 described above except for the operation of the electromagnetic coils 380 and 398 which is described in detail below.

The electromagnetic coil 380 is supported on the fixed jaw 340 and supports a first plunger 350. The electromagnetic coil 80 can be energized to advance the plunger 350 within the fixed jaw 340 and deenergized to retract the plunger 350 within the fixed jaw 340. Similarly, the electromagnetic coil 398 is supported on the rotating jaw 342 and supports a second plunger 352. The electromagnetic coil 98 can be energized to advance the plunger 352 within the rotating jaw 342 and deenergized to retract the plunger 352 within the rotating jaw 342.

When the tool assembly 316 is operated to stitch tissue, the electromagnetic coils 380, 398 are selectively energized and deenergized to advance and retract the plungers 350,352, respectively, to transfer the needle 306 between the jaws 340, 342. More specifically, when one of the electromagnets 380, 398 is energized, the respective plunger 350, 352 is advanced into engagement with the needle 306 to secure the needle 306 to the respective jaw 340, 342. In this respect, one of the electromagnetic coils 380, 398 is always energized and the other of the electromagnetic coils 380, 398 is always deenergized.

In some aspects of the disclosure, the plungers 350 and 352 can have a recessed distal end 354 that is configured to engage an outer contour of the needle 306. It is also envisioned that the needle 306 can include a notch 354 at each end of the needle 306 to receive the respective plunger 350, 352 to more securely hold the needle 306 within the respective jaw 340, 342.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:-
1. A stitching device comprising;
   a handle assembly including a switch and an actuator;
   an elongate body extending distally from the handle assembly and including a distal end portion; and
   a tool assembly supported on the distal end portion of the elongate body and including a fixed jaw, a rotating jaw, and a needle, the rotating jaw being movable in relation to the fixed jaw from a start position to a transfer position in response to operation of the actuator, the fixed jaw supporting a first electromagnetic coil and the rotating jaw supporting a second electromagnet coil, wherein each of the first and second electromagnetic coils can be independently energized via operation of the switch to secure the needle to one of the fixed jaw or the rotating jaw.
2. The stitching device of paragraph 1, wherein the first electromagnetic coil defines a first bore and the second electromagnetic coil defines a second bore, the needle including a first tapered end and a second tapered end, wherein in the start position, the first tapered end is positioned within the first bore or the second tapered end is positioned within the second bore, and in the transfer position, the first tapered end is positioned within the first bore and the second tapered end is positioned within the second bore.
3. The stitching device of paragraph 2, wherein the needle includes a curved body.
4. The stitching device of paragraph 3, wherein the needle is formed at least in part from a ferromagnetic material.
5. The stitching device of paragraph 4, wherein the first and second electromagnetic coils, when energized, create a magnetic field to retain the needle within a respective one of the bores of the first or second electromagnetic coils.
6. The stitching device of paragraph 1, wherein the first jaw defines a first bore that receives the first electromagnetic coil and the second jaw defines a second bore that receives the second electromagnetic coil, the first and second bores of the first and second electromechanical coils defining axes that are transverse to the longitudinal axis of the elongate body.
7. The stitching device of paragraph 2, further including a drive assembly including a drive rod having a distal end, a cam support member supported on the distal end of the drive rod, and a cam member that is supported on the cam support member, the drive rod movable between retracted and advanced positions in response to operation of the actuator, the rotating jaw including a helical cam slot that receives the cam member such that operation of the actuator causes movement of the rotating jaw in relation to the fixed jaw from the start position to the transfer position.
8. The stitching device of paragraph 1, wherein the first electromagnetic coils supports a first plunger and the second electromagnetic coil supports a second plunger, the first and second electromagnetic coils being operable to advance the first and second plungers, respectively, from a retracted position to an advanced position into engagement with the needle.
9. The stitching device of paragraph 8, wherein each of the first and second plungers defines a notch that is configured to receive the needle to retain the needle in engagement with a respective one of the respective first and second jaws.
10. The stitching device of paragraph 8, wherein the first electromagnetic coil defines a first bore and the second electromagnetic coil defines a second bore, the first and second bores defining axes that are parallel to the longitudinal axis of the elongate body.
11. The stitching device of paragraph 1, wherein each of the first and second electromagnetic coils is coupled to the switch by wires.
12. The stitching device of paragraph 1, wherein the actuator is positioned to engage the switch such that operation of the actuator activates the switch.
13. A tool assembly comprising:
   a fixed jaw, a rotating jaw, and a needle, the rotating jaw being movable in relation to the fixed jaw from a start position to a transfer position, the fixed jaw supporting a first electromagnetic coil and the rotating jaw supporting a second electromagnet coil, wherein each of the first and second coils can be independently energized to secure the needle to one of the fixed jaw or the rotating jaw.
14. The tool assembly of paragraph 13, wherein the first electromagnetic coil defines a first bore and the second electromagnetic coil defines a second bore, the needle including a first tapered end and a second tapered end, wherein in the start position, the first tapered end is positioned within the first bore or the second tapered end is positioned within the second bore, and in the transfer position, the first tapered end is positioned within the first bore and the second tapered end is positioned within the second bore.
15. The tool assembly of paragraph 14, wherein the needle is formed at least in part from a ferromagnetic material, wherein the first and second electromagnetic coils create a magnetic field when energized to retain the needle within a respective one of the bores of the first and second electromagnetic coils.
16. The tool assembly of paragraph 15, wherein the first and second bores of the first and second electromechanical coils define axes transverse to the longitudinal axis of the elongate body.
17. The tool assembly of paragraph 13, wherein the first electromagnetic coils supports a first plunger and the second electromagnetic coil supports a second plunger, the first and second electromagnetic coils being operable to advance the first and second plungers, respectively, from a retracted position to an advanced position into engagement with the needle.
18. The tool assembly of paragraph 17, wherein the first electromagnetic coil defines a first bore and the second electromagnetic coil defines a second bore, the first and second bores defining axes that are parallel to the longitudinal axis of the elongate body.
19. A stitching device comprising;
   a handle assembly including a switch and an actuator;
   an elongate body extending distally from the handle assembly;
   a drive assembly including a drive rod having a distal end, a cam support member supported on the distal end of the drive rod, and a cam member that is supported on the cam support member, the drive rod movable between retracted and advanced positions in response to operation of the actuator; and
   a tool assembly including a fixed jaw, a rotating jaw, and a needle, the rotating jaw including a helical cam slot that receives the cam member such that operation of the actuator causes movement of the rotating jaw in relation to the fixed jaw from a start position to a transfer position, the fixed jaw supporting a first electromagnetic coil and the rotating jaw supporting a second electromagnet coil, wherein each of the first and second electromagnetic coils can be energized via operation of the switch to secure the needle to one of the fixed jaw or the rotating jaw.
20. The tool assembly of paragraph 19, wherein the needle is formed at least in part from a ferromagnetic material, wherein the first and second electromagnetic coils create a magnetic field when energized to retain the needle within a respective one of the bores of the first and second electromagnetic coils.

## Claims

1. A stitching device comprising;
a handle assembly including a switch and an actuator;
an elongate body extending distally from the handle assembly and including a distal end portion; and
a tool assembly supported on the distal end portion of the elongate body and including a fixed jaw, a rotating jaw, and a needle, the rotating jaw being movable in relation to the fixed jaw from a start position to a transfer position in response to operation of the actuator, the fixed jaw supporting a first electromagnetic coil and the rotating jaw supporting a second electromagnet coil, wherein each of the first and second electromagnetic coils can be independently energized via operation of the switch to secure the needle to one of the fixed jaw or the rotating jaw.

2. The stitching device of claim 1, wherein the first electromagnetic coil defines a first bore and the second electromagnetic coil defines a second bore, the needle including a first tapered end and a second tapered end, wherein in the start position, the first tapered end is positioned within the first bore or the second tapered end is positioned within the second bore, and in the transfer position, the first tapered end is positioned within the first bore and the second tapered end is positioned within the second bore.

3. The stitching device of claim 2, wherein the needle includes a curved body.

4. The stitching device of claim 3, wherein the needle is formed at least in part from a ferromagnetic material.

5. The stitching device of claim 4, wherein the first and second electromagnetic coils, when energized, create a magnetic field to retain the needle within a respective one of the bores of the first or second electromagnetic coils.

6. The stitching device of any preceding claim, wherein the first jaw defines a first bore that receives the first electromagnetic coil and the second jaw defines a second bore that receives the second electromagnetic coil, the first and second bores of the first and second electromechanical coils defining axes that are transverse to the longitudinal axis of the elongate body.

7. The stitching device of claim 2, further including a drive assembly including a drive rod having a distal end, a cam support member supported on the distal end of the drive rod, and a cam member that is supported on the cam support member, the drive rod movable between retracted and advanced positions in response to operation of the actuator, the rotating jaw including a helical cam slot that receives the cam member such that operation of the actuator causes movement of the rotating jaw in relation to the fixed jaw from the start position to the transfer position.

8. The stitching device of any preceding claim, wherein the first electromagnetic coils supports a first plunger and the second electromagnetic coil supports a second plunger, the first and second electromagnetic coils being operable to advance the first and second plungers, respectively, from a retracted position to an advanced position into engagement with the needle; preferably wherein each of the first and second plungers defines a notch that is configured to receive the needle to retain the needle in engagement with a respective one of the respective first and second jaws.

9. The stitching device of claim 8, wherein the first electromagnetic coil defines a first bore and the second electromagnetic coil defines a second bore, the first and second bores defining axes that are parallel to the longitudinal axis of the elongate body.

10. The stitching device of any preceding claim, wherein each of the first and second electromagnetic coils is coupled to the switch by wires.

11. The stitching device of any preceding claim, wherein the actuator is positioned to engage the switch such that operation of the actuator activates the switch.

12. A tool assembly comprising:
a fixed jaw, a rotating jaw, and a needle, the rotating jaw being movable in relation to the fixed jaw from a start position to a transfer position, the fixed jaw supporting a first electromagnetic coil and the rotating jaw supporting a second electromagnet coil, wherein each of the first and second coils can be independently energized to secure the needle to one of the fixed jaw or the rotating jaw; preferably wherein the first electromagnetic coil defines a first bore and the second electromagnetic coil defines a second bore, the needle including a first tapered end and a second tapered end, wherein in the start position, the first tapered end is positioned within the first bore or the second tapered end is positioned within the second bore, and in the transfer position, the first tapered end is positioned within the first bore and the second tapered end is positioned within the second bore; preferably wherein the needle is formed at least in part from a ferromagnetic material, wherein the first and second electromagnetic coils create a magnetic field when energized to retain the needle within a respective one of the bores of the first and second electromagnetic coils.

13. The tool assembly of claim 12, wherein the first and second bores of the first and second electromechanical coils define axes transverse to the longitudinal axis of the elongate body; preferably wherein the first electromagnetic coils supports a first plunger and the second electromagnetic coil supports a second plunger, the first and second electromagnetic coils being operable to advance the first and second plungers, respectively, from a retracted position to an advanced position into engagement with the needle.

14. The tool assembly of claim 13, wherein the first electromagnetic coil defines a first bore and the second electromagnetic coil defines a second bore, the first and second bores defining axes that are parallel to the longitudinal axis of the elongate body.

15. A stitching device comprising;
a handle assembly including a switch and an actuator;
an elongate body extending distally from the handle assembly;
a drive assembly including a drive rod having a distal end, a cam support member supported on the distal end of the drive rod, and a cam member that is supported on the cam support member, the drive rod movable between retracted and advanced positions in response to operation of the actuator; and
a tool assembly including a fixed jaw, a rotating jaw, and a needle, the rotating jaw including a helical cam slot that receives the cam member such that operation of the actuator causes movement of the rotating jaw in relation to the fixed jaw from a start position to a transfer position, the fixed jaw supporting a first electromagnetic coil and the rotating jaw supporting a second electromagnet coil, wherein each of the first and second electromagnetic coils can be energized via operation of the switch to secure the needle to one of the fixed jaw or the rotating jaw; preferably wherein the needle is formed at least in part from a ferromagnetic material, wherein the first and second electromagnetic coils create a magnetic field when energized to retain the needle within a respective one of the bores of the first and second electromagnetic coils.
